# EUROPEAN PATENT APPLICATION

(11) **EP 3 949 895 A1**
(43) Date of publication of application: **09.02.2022**
(21) Application number: 19923359.4
(22) Date of filing: 30.10.2019
(51) Int. Cl.: A61C 8/00, A61C 13/00, A61C 9/00, A61B 34/10

(54) **CUSTOM IMPLANT FIXTURE, METHOD FOR MANUFACTURING SAME, AND CUSTOM IMPLANT SYSTEM USING SAME**

(30) Priority: 29.03.2019 KR 20190036667
(71) Applicant: Dentium Co., Ltd., Gangnam-gu Seoul 06373 (KR)
(72) Inventor: CHUNG, Sung Min, Suwon-si Gyeonggi-do 16229 (KR); LEE, Ki Sun, Seoul 06277 (KR); SHIN, Sang Wan, Yongin-si, Gyeonggi-do 17005 (KR)
(74) Representative: von Bülow & Tamada
(86) International application number: PCT/KR2019/014473
(87) International publication number: WO 2020/204294

(57) **Abstract**

A personalized implant fixture may include: a first fixture portion having a first cross section determined by a shape of a tooth to be extracted covered by an alveolar bone; and a second fixture portion formed to extend from the first fixture portion, determined from a position and a direction of a root part of the tooth with respect to the alveolar bone, and having a second cross section.

## Description

### [Technical Field]

The present invention relates to a personalized implant fixture having a shape of a natural dental root, a manufacturing method thereof, and a personalized implant system using the same.

### [Background Art]

An implant in dentistry refers to a treatment in which an artificially manufactured tooth is placed in a part where a tooth is lost to make it look like that a tooth exists. In a general implant method, instead of a lost tooth, a fixture made of an osseointegration material is implanted into an alveolar bone through surgery, and an artificial tooth, which is a prosthesis, is fixed onto an upper portion of the fixture.

A general method of placing the fixture is to form a rotationally symmetrical hole in the alveolar bone by cutting the alveolar bone and then insert an implant of an appropriate size into the rotationally symmetrical hole. Rotational symmetric implant fixtures manufactured to have various shapes, diameters and lengths are integrated with the alveolar bone when several weeks have elapsed after placement.

In such a dental implant procedure, in a case where an implant should be placed immediately after tooth loss (immediately after tooth extraction), cross-sectional shapes of a tooth socket remaining after the tooth extraction and a ready-made implant are not matched to each other, and thus, the implant within the tooth socket is not sufficiently fixed. Therefore, it is generally accepted as a stable procedure to perform an implant procedure after the bone is first recovered after the tooth extraction.

However, after the tooth extraction, the tooth socket causes loss of a height and a width of alveolar bone in a spontaneous cure process, which significantly affects the subsequent implant placement and prognosis. Accordingly, a technology of placing various bone graft materials and using a shielding film in order to fill the empty space between the tooth socket and the implant in a case where the implant is placed immediately after the tooth extraction in order to maintain a form of the alveolar bone before the tooth extraction has been proposed. For example, International Patent Publication No. WO2017/007248 proposes to use a personalized scaffold graft material suitable for a form of a tooth socket, and International Patent Publication No. WO2007/038817 introduces a method of using a personalized implant having a form of a dental root.

However, in a case where an implant fixture having a rotational symmetrical object as in the related art is placed in the tooth socket, an empty space is generated between the tooth socket and the implant, such that it is difficult to predict an initial fixing force and a change in an appearance of the alveolar bone. In addition, in a case where the bone graft material and the shielding film are used, a considerable cure period such as several months or more is required for integration between the bone graft material and the surrounding bone implant.

In addition, an existing dental root-type implant has a disadvantage that an initial fixing force cannot but be weak because a pressure is applied to the implant fixture to place the implant fixture in the tooth socket without additional bone cutting. In particular, in a case where tooth extraction is performed due to severe periodontitis, a depth of the alveolar bone is very low, such that it is difficult to directly apply such an existing dental root-type implant. As a result, the existing dental root-type implant may be used only in an extremely limited case such as a case of tooth extraction in a state where the alveolar bone is healthy. In addition, in a case of an upper prosthesis, a technology for a connection method has not been suggested, and in a case where the upper prosthesis is expected in a manufacturing design form, it is expected that only a form of an adhesion-type prosthesis will be able to be used. Furthermore, since a detailed technology for a method for manufacturing a shape of the dental root-type implant has not been suggested, concreteness of a method of putting the existing dental root-type implant to practical use is low, such that there are rare examples in which a procedure of the existing dental root-type implant is actually performed.

### Related Art Document

International Patent Publication No. WO2017/007248 (published on January 12, 2017)
International Patent Publication No. WO2007/038817 (published on April 12, 2007)

### [Disclosure]

### [Technical Problem]

An object of the present invention is to provide a personalized implant fixture that has a personalized shape accurately fit for a tooth socket by minimizing a gap between the tooth socket and an implant even in a case where the implant is immediately placed after tooth extraction and is capable of increasing an initial fixing force by cutting a healthy alveolar bone of a lower portion of the tooth socket and fastening a corresponding cut space and the implant to each other, instead of a contaminated alveolar bone of an upper portion of the tooth socket due to dental trauma, dental caries, periodontitis, or the like, before the tooth extraction, a manufacturing method thereof, and a personalized implant system using the same.

### [Technical Solution]

According to an embodiment of the present invention, a personalized implant fixture may include: a first fixture portion having a first cross section determined by a shape of a tooth to be extracted covered by an alveolar bone; and a second fixture portion formed to extend from the first fixture portion, determined from a position and a direction of a root part of the tooth with respect to the alveolar bone, and having a second cross section.

As an example according to the present invention, the first cross section may be an asymmetric cross section of a natural outer surface of the tooth.

As an example according to the present invention, the second cross section may be a circular cross section inserted into a circular hole obtained by drilling the alveolar bone.

As an example according to the present invention, the second fixture portion may be formed in a cylindrical or conical shape so as to be directly inserted into the alveolar bone by a non-rotating method.

As an example according to the present invention, the first fixture portion may further include a first surface shape portion for increasing an osseointegration bonding force.

As an example according to the present invention, the second fixture portion may further include a second surface shape portion for increasing an initial fixing force.

As an example according to the present invention, the first surface shape portion and the second surface shape portion may be formed in a thread or irregularity shape.

As an example according to the present invention, a fastening shape portion for fixing an abutment may be formed on an upper portion of the first fixture portion.

As an example according to the present invention, an upper prosthesis exposed to the outside of the alveolar bone may be directly attached to an upper portion of the first fixture portion.

According to another embodiment of the present invention, a manufacturing method of a personalized implant fixture may include: receiving tomography data information of the head and neck; generating a three-dimensional model including an alveolar bone and a tooth to be extracted through the data information; virtually removing a tooth of a part to be extracted from the three-dimensional model; and designing a personalized implant fixture to be placed in a tooth socket formed by removing the tooth, wherein the personalized implant fixture includes: a first fixture portion having a first cross section determined by a shape of the tooth to be extracted covered by the alveolar bone; and a second fixture portion formed to extend from the first fixture portion, determined from a position and a direction of a root part of the tooth with respect to the alveolar bone, and having a second cross section.

As an example according to the present invention, the manufacturing method of a personalized implant fixture may further include, after the designing of the personalized implant to be placed in the tooth socket formed by removing the tooth, designing a surgical guide used for placing the personalized implant.

As an example according to the present invention, the personalized implant fixture and the surgical guide may be manufactured by 3D printing.

As an example according to the present invention, the first cross section may be an asymmetric cross section of a natural outer surface of the tooth, and the second cross section may be a circular cross section inserted into a circular hole obtained by drilling the alveolar bone.

According to still another embodiment of the present invention, a personalized implant system may include: a data obtaining unit receiving tomography data information of the head and neck; a three-dimensional modeling unit generating a three-dimensional model including an alveolar bone and a tooth to be extracted through the data information; an implant design generating unit virtually removing a tooth of a part to be extracted from the three-dimensional model and generating design of a personalized implant fixture to be placed in a tooth socket formed by removing the tooth; a guide design generating unit generating a design of a surgical guide used for placing the personalized implant fixture; and a machining unit manufacturing the personalized implant fixture and the surgical guide by 3D printing or milling, wherein the personalized implant fixture includes: a first fixture portion having a first cross section determined by a shape of the tooth to be extracted covered by the alveolar bone; and a second fixture portion formed to extend from the first fixture portion, determined from a position and a direction of a root part of the tooth with respect to the alveolar bone, and having a second cross section.

### [Advantageous Effects]

With the personalized implant fixture according to the present invention including the first fixture portion having the first cross section determined by the shape of the tooth to be extracted covered by the alveolar bone and the second fixture portion formed to extend from the first fixture portion, determined from the position and the direction of the root part of the tooth with respect to the alveolar bone, and having the second cross section, an initial safety of an implant immediately after tooth extraction may be secured through implant placement according to an alveolar bone drilling method according to a general implant placement technique, and the implant may be accurately placed in the tooth socket.

In a case of the personalized implant according to the present invention, even in a case where absorption of a tooth socket surrounding bone significantly progresses as periodontal disease progresses, the second fixture portion is inserted and fixed into a hole formed by drilling, and an initial fixing force after placement of the personalized implant that may not be achieved by an existing natural tooth extraction-type implant having a simple dental root form may thus be secured, such that a target to which the personalized implant is to be applied may be expanded.

### [Description of Drawings]

FIGS. 1A and 1B are views illustrating a case of a healthy tooth and a case of a tooth with periodontal disease according to the present invention, respectively.

FIGS. 2A and 2B are conceptual comparative cross-sectional views illustrating appearances of a tooth without periodontal disease before being extracted and an implant placed in a state in which the tooth is extracted according to the present invention, respectively.

FIGS. 3A and 3B are conceptual comparative cross-sectional views illustrating appearances of a tooth with periodontal disease before being extracted and an implant placed in a state in which the tooth is extracted according to the present invention, respectively.

FIGS. 4I to 4IV are cross-sectional views taken along lines I-I and II-II of FIG. 2 and cross-sectional views taken along lines III-III and IV-IV of FIG. 3.

FIGS. 5A to 5G are schematic cross-sectional views illustrating various embodiments of a surface shape portion of a personalized implant fixture according to the present invention.

FIGS. 6A to 6D are conceptual cross-sectional views sequentially illustrating manufacturing processes of the personalized implant fixture according to the present invention.

FIG. 7 is a flowchart for describing a manufacturing method of a personalized implant fixture according to the present invention.

### [Best Mode for Invention]

Hereinafter, a personalized implant fixture and a manufacturing method thereof according to the present invention will be described in detail with reference to the accompanying drawings.

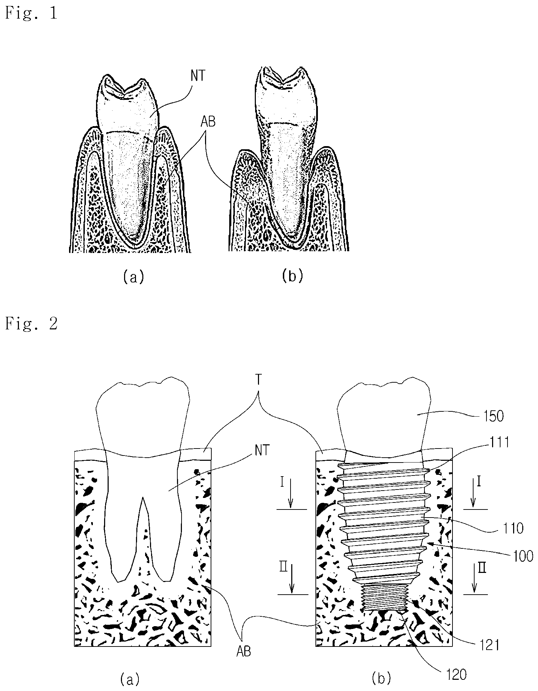

FIGS. 1A and 1B are views illustrating a case of a healthy tooth and a case of a tooth with periodontal disease according to the present invention, respectively. Unlike a case of a healthy tooth (FIG. 1A), in a case of a patient with severely advanced periodontitis (FIG. 1B), a height of an alveolar bone is low and an alveolar bone AB surrounding a natural tooth NT is also in a weakened state. Even in a case where an implant is immediately placed in the same shape as a shape of the tooth NT after the tooth is extracted in this state, a depth of the alveolar bone covering the extracted tooth NT is shallow and an area of the alveolar bone covering the extracted tooth NT is small, and it is thus difficult to secure an initial fixing force of the placed implant. The personalized implant fixture according to the present invention provides an initial fixing force that may be applied even in a case where the depth of the alveolar bone AB is low as described above.

FIGS. 2A and 2B are conceptual comparative cross-sectional views illustrating appearances of a tooth without periodontal disease before being extracted and an implant placed in a state in which the tooth is extracted according to the present invention, respectively.

As illustrated in FIGS. 2A and 2B, in a case where there is a tooth NT with bifurcated roots (FIG. 2A), an example of a personalized implant fixture 100 immediately placed instead of the extracted tooth NT is illustrated in FIG. 2B.

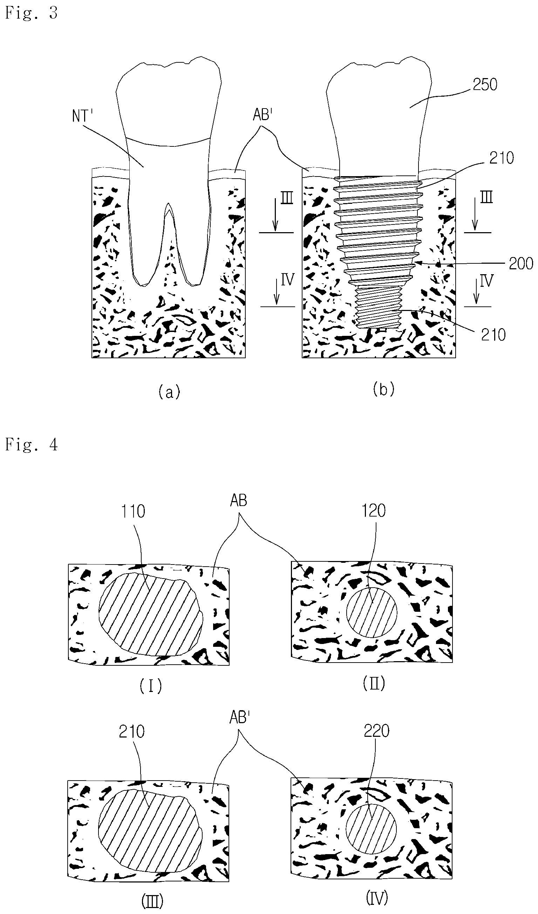

The personalized implant fixture 100 has a first fixture portion 110 having a first cross section determined by a shape of the tooth NT to be extracted covered by an alveolar bone AB, and a second fixture portion 120 formed to extend from the first fixture portion 110. As illustrated in FIG. 4I, a first cross section of the first fixture portion 110 is an asymmetric cross section of a natural outer surface of the tooth NT. In this case, there is almost no empty space between an outer peripheral surface of the first fixture portion 110 and a tooth socket of the alveolar bone AB. The second fixture portion 120 is determined from a position and a direction of a root part of the tooth NT with respect to the alveolar bone AB at a lower end of the first fixture portion 110, and has a second cross section. As illustrated in FIG. 4II, the second cross section is a circular cross section that may be inserted into a circular hole obtained by drilling the alveolar bone AB.

The second fixture portion 120 may be placed slightly deeper than roots of the natural tooth NT. This means that it may contribute to securing an initial fixing force of the personalized implant fixture in a case where a depth of the tooth socket (TS) is shallow as periodontitis significantly progresses.

The second fixture portion 120 is fixed to a drilling hole by a non-rotating method, that is, a direct insertion or press-fit method rather than a method by a screw with respect to the alveolar bone AB. Unlike the first fixture portion 110 having an asymmetric or irregular shape, the first fixture portion 110 may be formed in a cylindrical or conical shape to be easily inserted into the drilling hole.

The first fixture portion 110 and the second fixture portion 120 may be formed of a material having characteristics of osseointegration, including titanium and a titanium alloy.

A first surface shape portion 111 for increasing an osseointegration bonding force may be formed on a surface of the first fixture portion 110, and a second surface shape portion 121 for increasing the initial fixing force may also be formed on a surface of the second fixture portion 120. The first surface shape portion 111 and the second surface shape portion 121 may be formed in a thread or irregularity shape to increase a bonding surface area at the time of integration with the alveolar bone AB.

In addition, surface treatment may be applied to the first surface shape portion 111 and the second surface shape portion 121 so as to promote the integration with the alveolar bone AB. Examples of such surface treatment include a method of forming fine irregularities by hitting sand particles absorbed by a metal using strong pressure (resorbable blast media (RBM)), a method of forming finer crater structures in the irregularities by treating a surface of an RBM with an acid solution (sandblasted and acid etched (SA)), a method of hitting aluminum oxide particles that are not absorbed by a metal with a strong pressure and then performing acid solution treatment (sandblasted, large-grit acid etched (SLA)), a method of coating hydroxyapatite, which is a bone matrix component, on a surface of a fixture (hydroxyapatite coating (HA)), and the like.

FIGS. 3A and 3B are conceptual comparative cross-sectional views illustrating appearances of a tooth with periodontal disease before being extracted and an implant placed in a state in which the tooth is extracted according to the present invention, respectively. In the present example, actually, a depth of a tooth NT' embedded in an alveolar bone AB' is relatively low due to periodontal disease, and it may thus be relatively difficult for an implant fixture 200 having a natural dental root shape to satisfy a depth for fixation. To this end, the personalized implant fixture 200 also has a second fixture portion 220 together with the first fixture portion 210 as in the previous example, the first fixture portion 110 has an asymmetric first cross section having the same shape as a tooth shape determined by a shape of the toot NT' to be extracted covered by the alveolar bone AB', and the second fixture portion 220 also has a circular second cross section determined from a position and a direction of a root portion of the tooth NT' with respect to the alveolar bone AB' (see FIGS. 4III and 4IV).

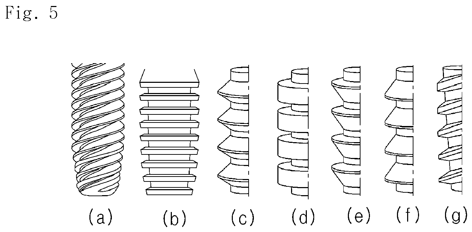

FIGS. 5A to 5G are schematic cross-sectional views illustrating various embodiments of a surface shape portion of a personalized implant fixture according to the present invention. As described above, the first fixture portions 110 and 210 and the second fixture portions 120 and 220 may have various surface shape portions for improving osseointegration characteristics with the alveolar bone. FIG. 5A illustrates that the surface shape portion is formed in a thread shape, and may have horizontal repetitive irregularities as illustrated in FIG. 5B. In addition, the osseointegration characteristics may be improved by making angles forming threads or irregularities different from each other or diversifying geometric shapes of the threads or the irregularities, as illustrated in FIGS. 5C to 5G. In addition, the surface shape portions may have grooves or irregularities at positions spaced apart from each other along circumferential surfaces of the first and second fixture portions 110 and 210 and the second fixture portions 120 and 220 or have a change in position according to a height.

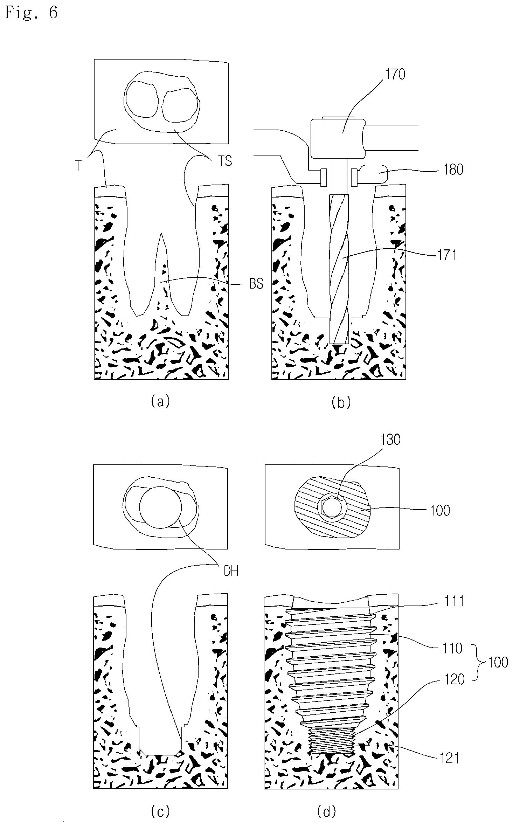

FIGS. 6A to 6D are conceptual cross-sectional views sequentially illustrating manufacturing processes of the personalized implant fixture according to the present invention. In the present example, a process of placing the personalized implant fixture 100 after the tooth NT is extracted in a state as illustrated in FIG 2A is illustrated. Lower drawings of two drawings of each of FIGS. 6A to 6D are longitudinal sectional views of a tooth socket TS and an alveolar bone AB, and upper drawings thereof are schematic plan views.

As illustrated in FIG. 6A, the tooth socket TS is formed at a location where the tooth NT is extracted, and an implant basically having an appearance similar to an appearance corresponding to the tooth socket TS is manufactured by a manufacturing method to be described later, and fills the tooth socket TS, in order to immediately place the implant.

To this end, first, as illustrated in FIG. 6B, an alveolar bone BS of a middle part between a root and the other root of the tooth NT that are bifurcated is removed. Even in a case where the alveolar bone BS of the middle part is maintained, osseointegration is not densely achieved, but the bone may not be filled due to the alveolar bone BS of the middle part, and it may thus be more effective to remove the alveolar bone BS of the middle part.

Next, a drilling hole DH is formed in the deepest part of the tooth socket TS through a handpiece 170 and a drill 171. Such a drilling hole DH serves as an entire reference guide so that the first and second fixture portions 110 and 120 formed integrally with each other may be inserted in an axial direction, and furthermore, makes it possible to secure a fixing force through contact with the second fixture portion 120 and osseointegration. A surgical guide 180 suitable for the tooth socket TS may be used in order to form the drilling hole DH.

When the drilling hole DH is formed, the personalized implant fixture 100 having the first fixture portion 110 and the second fixture portion 120 is immediately inserted into the drilling hole DH. In this case, an upper prosthesis 150 (see FIG. 2) exposed to the outside of the alveolar bone AB is directly attached to an upper portion of the second fixture portion 120, or a fastening shape portion 130 for fixing an abutment or the upper prosthesis 150 may be formed on the upper portion of the second fixture portion 120 as illustrated in the upper drawing of FIG. 5D. Before the upper prosthesis 150 is attached or fastened, a healing period may be applied so that the personalized implant fixture 100 may secure a fixing force.

A personalized implant system according to the present invention may include a data obtaining unit, a three-dimensional modeling unit, an implant design generating unit, a guide design generating unit, a machining unit, and the like.

The data obtaining unit receives data information by tomography such as computerized tomography (CT) of the head and neck. The three-dimensional modeling unit generates a three-dimensional model including an alveolar bone and a tooth to be extracted through such data information. When the three-dimensional model is generated, the implant design generating unit virtually removes a tooth of a part to be extracted from the three-dimensional model, and generates a design of a personalized implant fixture to be placed in a tooth socket formed by removing the tooth. In addition, the guide design generating unit generates a design of a dedicated surgical guide used for the placement of such a personalized implant. The machining unit manufactures the personalized implant fixture and the surgical guide by 3D printing or milling. Here, the personalized implant fixture includes the first fixture portion 110 having the first cross section and the second fixture portion 120 having the second cross section described above in common.

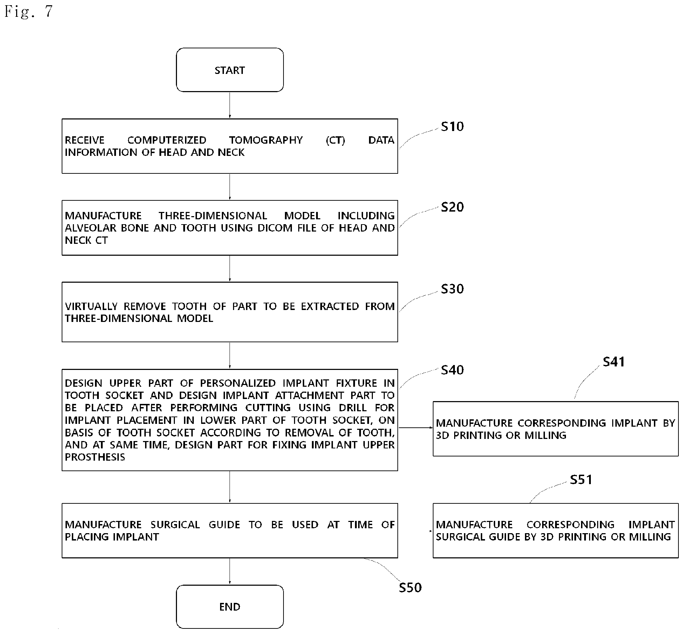

A manufacturing method of a personalized implant fixture according to the present invention through these equipment or elements will be described. FIG. 7 is a flowchart for describing a manufacturing method of a personalized implant fixture according to the present invention.

First, tomography data information of a patient's head and neck is received by the data obtaining unit (S10). The received data information is tomography image information such as CT.

Next, the three-dimensional modeling unit manufactures a three-dimensional model including the alveolar bone and the tooth using a DICOM file of the head and neck CT (S20).

Through this, the implant design generating unit virtually removes a tooth of a part to be extracted from the three-dimensional model (S30), and the first fixture portion 110, which is an upper part of the personalized implant fixture, is designed in the tooth socket and the second fixture portion 120, which is an implant attachment part to be placed after performing cutting using a drill for implant placement, is designed in a lower part of the tooth socket, on the basis of the tooth socket according to the removal of the tooth (S40). The guide design generating unit is provided separately from or integrated with the implant design generating unit to generate a design of a part for fixing an implant upper prosthesis and a design of the surgical guide to be used at the time of implant placement (S50).

The machining unit may manufacture the personalized implant fixture whose design has been completed by 3D printing or milling (S41), and may also manufacture the surgical guide by 3D printing or milling (S51).

The personalized implant fixture and the surgical guide manufactured as described above are manufactured and prepared before tooth extraction to allow surgery to be performed immediately after the tooth extraction, such that the personalized implant is directly placed.

The configurations and the methods of the described embodiments of the personalized implant fixture, the manufacturing method thereof, and the personalized implant system using the same described above are not restrictively applied. All or some of the respective exemplary embodiments may be selectively combined with each other so that the exemplary embodiments described above may be variously modified.

## Claims

1. A personalized implant fixture comprising:
a first fixture portion having a first cross section determined by a shape of a tooth to be extracted covered by an alveolar bone; and
a second fixture portion formed to extend from the first fixture portion, determined from a position and a direction of a root part of the tooth with respect to the alveolar bone, and having a second cross section.

2. The personalized implant fixture of claim 1, wherein the first cross section is an asymmetric cross section of a natural outer surface of the tooth.

3. The personalized implant fixture of claim 1, wherein the second cross section is a circular cross section inserted into a circular hole obtained by drilling the alveolar bone.

4. The personalized implant fixture of claim 1, wherein the second fixture portion is formed in a cylindrical or conical shape so as to be directly inserted into the alveolar bone by a non-rotating method.

5. The personalized implant fixture of claim 1, wherein the first fixture portion further includes a first surface shape portion for increasing an osseointegration bonding force.

6. The personalized implant fixture of claim 1, wherein the second fixture portion further includes a second surface shape portion for increasing an initial fixing force.

7. The personalized implant fixture of claim 5 or 6, wherein the first surface shape portion and the second surface shape portion are formed in a thread or irregularity shape.

8. The personalized implant fixture of claim 1, wherein a fastening shape portion for fixing an abutment is formed on an upper portion of the first fixture portion.

9. The personalized implant fixture of claim 1, wherein an upper prosthesis exposed to the outside of the alveolar bone is directly attached to an upper portion of the first fixture portion.

10. A manufacturing method of a personalized implant fixture, comprising:
receiving tomography data information of the head and neck;
generating a three-dimensional model including an alveolar bone and a tooth to be extracted through the data information;
virtually removing a tooth of a part to be extracted from the three-dimensional model; and
designing a personalized implant fixture to be placed in a tooth socket formed by removing the tooth,
wherein the personalized implant fixture includes:
a first fixture portion having a first cross section determined by a shape of the tooth to be extracted covered by the alveolar bone; and
a second fixture portion formed to extend from the first fixture portion, determined from a position and a direction of a root part of the tooth with respect to the alveolar bone, and having a second cross section.

11. The manufacturing method of a personalized implant fixture of claim 10, further comprising, after the designing of the personalized implant to be placed in the tooth socket formed by removing the tooth, designing a surgical guide used for placing the personalized implant.

12. The manufacturing method of a personalized implant fixture of claim 11, wherein the personalized implant fixture and the surgical guide are manufactured by 3D printing.

13. The manufacturing method of a personalized implant fixture of claim 10, wherein the first cross section is an asymmetric cross section of a natural outer surface of the tooth, and
the second cross section is a circular cross section inserted into a circular hole obtained by drilling the alveolar bone.

14. A personalized implant system comprising:
a data obtaining unit receiving tomography data information of the head and neck;
a three-dimensional modeling unit generating a three-dimensional model including an alveolar bone and a tooth to be extracted through the data information;
an implant design generating unit virtually removing a tooth of a part to be extracted from the three-dimensional model and generating design of a personalized implant fixture to be placed in a tooth socket formed by removing the tooth;
a guide design generating unit generating a design of a surgical guide used for placing the personalized implant; and
a machining unit manufacturing the personalized implant fixture and the surgical guide by 3D printing or milling,
wherein the personalized implant fixture includes:
a first fixture portion having a first cross section determined by a shape of the tooth to be extracted covered by the alveolar bone; and
a second fixture portion formed to extend from the first fixture portion, determined from a position and a direction of a root part of the tooth with respect to the alveolar bone, and having a second cross section.
